## Europäisches Patentamt
## European Patent Office
## Office européen des brevets

(19)

(11) Veröffentlichungsnummer : **0 071 730**
**B1**

(12)

# EUROPÄISCHE PATENTSCHRIFT

(45) Veröffentlichungstag der Patentschrift :
19.12.84

(51) Int. Cl.³ : **C 12 Q   1/28**

(21) Anmeldenummer : 82105552.2

(22) Anmeldetag : 23.06.82

(54) Stabilisiertes Reagenz zum Nachweis von Wasserstoffperoxid.

(30) Priorität : 23.06.81 DE 3124590

(43) Veröffentlichungstag der Anmeldung :
16.02.83 Patentblatt 83/07

(45) Bekanntmachung des Hinweises auf die Patenterteilung : 19.12.84 Patentblatt 84/51

(84) Benannte Vertragsstaaten :
AT BE CH DE FR GB IT LI LU NL SE

(56) Entgegenhaltungen :
EP-A- 0 007 787
EP-A- 0 036 563
EP-A- 0 054 358
DE-A- 2 600 526
DE-A- 2 843 539
DE-A- 2 925 365
US-E-    29 498

(73) Patentinhaber : BOEHRINGER MANNHEIM GMBH
Patentabteilung, Abt. E Sandhofer Strasse 112-132
Postfach 31 01 20
D-6800 Mannheim 31 Waldhof (DE)

(72) Erfinder : Klose, Sigmar, Dr.
Breitenloh 7
D-8131 Berg 2 (DE)
Erfinder : Schlumberger, Helmut
Kaiser-Heinrich-Strasse 23
D-8121 Polling (DE)

(74) Vertreter : Weickmann, Heinrich, Dipl.-Ing. et al
Patentanwälte Dipl.-Ing. H.Weickmann Dipl.-Phys.Dr.
K.Fincke Dipl.-Ing. F.A.Weickmann Dipl.-Chem. B.
Huber Dr.-Ing. H. Liska Dipl.-Phys.Dr. J. Prechtel
Postfach 860820
D-8000 München 86 (DE)

## Beschreibung

Die Erfindung betrifft ein Reagenz zum Nachweis von $H_2O_2$ oder $H_2O_2$ liefernden Systemen mit überlegener Stabilität, insbesondere in Form der wässrigen Lösung.

Aus DE-OS 28 43 539 ist ein Testmittel zum Bestimmen von oxidierenden Substanzen bekannt, das aus einem Hydrazon und einem Hydroxynaphthalinsulfonat besteht. Weiterhin ist aus DE-OS 29 25 365 ein Testmittel zur Bestimmung von Harnsäure bekannt, das neben Naphthol oder Phenol und 4-Aminophenazon Ferrocyanidionen enthält. Diese beiden Testsysteme arbeiten auf Basis von Phenolen bzw. Naphtholen. Der Ersatz des Alkohols durch ein aromatisches Amin verschlechtert die Reproduzierbarkeit.

Reagenzien zum Nachweis von $H_2O_2$ oder $H_2O_2$ liefernden Systemen, welche die unter Farbstoffbildung verlaufende oxidative Kupplung zwischen Methylbenzthiazolonhydrazon oder einem Derivat davon mit einem aromatischen Amin, die durch $H_2O_2$ hervorgerufen wird, ausnutzen, sind bekannt. Ein Vorteil dieser Reagenzien ist darin zu sehen, daß der gebildete Farbstoff hinsichtlich seiner Menge und dementsprechend auf der Farbintensität der $H_2O_2$-Menge proportional ist und durch Bestimmung der Farbtiefe nach dem Beerschen Gesetz die quantitative $H_2O_2$-Bestimmung ermöglicht. Ein weiterer Vorteil dieses Reagenz besteht darin, daß es auch zusammen mit $H_2O_2$ liefernden Systemen, insbesondere enzymatischen Systemen, eingesetzt werden kann. Derartige $H_2O_2$ liefernde enzymatische Systeme bestehen in der Regel aus einer Oxidase, welche ein zu bestimmendes Substrat unter Bildung von $H_2O_2$ in gepufferter Lösung oxidiert. Das gebildete $H_2O_2$ bewirkt dann in Gegenwart von Peroxidase die oben erwähnte oxidative Kupplung unter Farbstoffbildung. Ein erheblicher Nachteil des bekannten Reagenz besteht jedoch in seiner Instabilität. Die Kupplungskomponenten neigen zur Autoxidation unter Farbstoffbildung. Dieser Nachteil ist besonders ausgeprägt bei den gebrauchsfertigen wässrigen Lösungen dieser Reagenzien. Aber auch die Trockenform des Reagenz läßt hinsichtlich der Stabilität zu wünschen übrig.

Der Erfindung liegt daher die Aufgabe zugrunde, den erwähnten Nachteil unter Aufrechterhaltung der angegebenen Vorteile zu beseitigen und ein Reagenz der eingangs erwähnten Art zu schaffen, welches hinsichtlich der Autoxidationsempfindlichkeit wesentlich verbessert ist. Eine weitere Aufgabe der Erfindung besteht darin, die Linearität der Farbstoffbildung in Abhängigkeit von der $H_2O_2$-Menge zu verbessern.

Gelöst wird diese Aufgabe erfindungsgemäß durch ein stabilisiertes Reagenz zum Nachweis von $H_2O_2$ oder $H_2O_2$ liefernden Systemen auf Basis von Methylbenzthiazolonhydrazon oder einem Derivat desselben als Farbbildner und eines damit zur oxidativen Kupplung unter Farbentwicklung befähigten aromatischen Amins, enthaltend Puffersubstanz und Peroxidase, welches gekennzeichnet ist durch einen Gehalt an wenigstens einer Substanz aus der Gruppe Alkaliferrozyanid, Chelatbildner und Alkaliazid als Stabilisator sowie gegebenenfalls einer Oxidase.

Der Farbbildner besteht beim erfindungsgemäßen Reagenz vorzugsweise aus Methylbenzthiazolonhydrazonsulfonat, (MBTH-S) welches sich von Methylbenzthiazolonhydrazon (MBTH) durch eine Sulfonatgruppe am aromatischen Ring unterscheidet.

Als aromatische Amin-Kupplungskomponente eignen sich prinzipiell alle aromatischen Verbindungen, welche chinoide Strukturen zu bilden vermögen und durch Halogenatome, Niedrigalkylgruppen, Hydroxyalkylgruppen oder/und Sulfonsäuregruppen substituiert sind. Eine Aufzählung geeigneter Amine findet sich in Analytical Chemistry *33*, 723-724 (1961). Daneben können auch phenolische amino-verbindungen als Kupplungskomponenten verwendet werden, beispielsweise Aminonaphtholdisulfonsäure. Bevorzugt werden im Rahmen der Erfindung m-Toluidine eingesetzt, insbesondere Ethylhydroxytoluidin oder N-Ethyl-N-(2-Sulfo-ethyl)-m-toluidin.

Als Alkaliferrozyanid können die Natrium-, Kalium-, Lithium- und Ammoniumsalze verwendet werden. Bevorzugt wird das Kaliumsalz.

Als Chalatbildner kommen die bekannten, zur Komplexbildung befähigten Substanzen dieser Art in Betracht. Bevorzugt werden Aminpolyazetate wie z. B. Ethylendiamintetraessigsäure oder deren Natriumsalz, Nitrilotriessigsäure, N-Hydroxyethylendinitrilotetraessigsäure, Cyklohexandiamintetraessigsäure, Cyklohexylendinitrilotetraessigsäure, Diäthylentriaminpentaessigsäure und 3,6-Dioxaoctamethylendinitrilotetraessigsäure. Besonders bevorzugt wird das Ethylendiamintetraessigsäurenatriumsalz.

Die Wirkungsweise des Chelatbildners ist nicht bekannt. Die erwähnte unerwünschte Autoxidationsreaktion verläuft offenbar nicht unter dem katalytischen Einfluß von Schwermetallionen, die durch den Chelatbildner in unschädliche Komplexe überführt wurden, da die Autoxidationsreaktion durch den Zusatz katalytischer Mengen von Schwermetallionen an sich nicht beeinflußt wird.

Als Alkaliazid wird Natriumazid bevorzugt, jedoch können auch die Kalium-, Lithium- und Ammoniumsalze verwendet werden.

Die besten Ergebnisse werden mit einem erfindungsgemäßen Reagenz erhalten, welches Alkaliferrozyanid, Chelatbildner und Alkaliazid gleichzeitig enthält. In einer bevorzugten Zusammensetzung enthält das Reagenz, bezogen auf einen Liter gebrauchsfertige wässrige Reagenzlösung, 1 bis 500 mg Ferrozyanid, 1 bis 1 000 mg Ethylendiamintetraazetat und 5 bis 500 mg Alkaliazid.

2

Die Konzentration an Farbbildner liegt zweckmäßig zwischen 0,01 und 5 mMol/l, die Menge an aromatischem Amin zwischen 0,01 und 10 mMol/l. Besonders bevorzugt wird eine Zusammensetzung, die 10 bis 50 mg Ferrocyanid, 100 bis 1 000 mg Ethylendiamintetraazetat und 50 bis 500 mg Alkaliazid enthält.

Als Puffersubstanz eignen sich solche, die im etwa neutralen Bereich, d. h. zwischen etwa pH 5,0 und 9,0 puffern. Geeignete Konzentrationen liegen im allgemeinen zwischen 0,02 und 0,5 mol/l. Eine geeignete Peroxidasemenge liegt zwischen 0,005 und $10 \times 10^3$ U/l.

Das erfindungsgemäße Reagenz eignet sich auch zur Aufbringung auf saugfähiges Trägermaterial, beispielsweise Papier, poröse Kunstoffolien, poröses Glasgranulat und dergleichen. Bevorzugte Träger sind solche, wie sie für Teststreifen bekannt sind. Der $H_2O_2$-Nachweis kann in diesem Falle qualitativ durch Auftropfen der zu untersuchenden Probelösung auf den das erfindungsgemäße Reagenz enthaltenden blatt- oder bandförmigen Träger oder durch Einbringen des Trägermaterials in bestimmter Menge in die zu untersuchende Lösung erfolgen. Im letzteren Falle kann eine quantitative Bestimmung durchgeführt werden in gleicher Weise wie beim trägerlosen Reagenz.

Wie bereits erwähnt, eignet sich das erfindungsgemäße Reagenz auch zur Bestimmung von $H_2O_2$-liefernden Systemen. Hierunter werden Verbindungen verstanden, die in einer Vorreaktion unter Bildung von $H_2O_2$ stöchiometrisch umgesetzt werden. Typische Beispiele sind die durch Oxidasen quantitativ umgesetzte Substanzen wie z. B. Glucose, Cholesterin, Harzsäure, Glycerin, Aminosäuren, Xanthin, Lactat, und dergleichen. In diesem Falle enthält das erfindungsgemäße Reagenz zusätzlich noch die entsprechende Oxidase, im Falle der Harnsäure also beispielsweise Uricase.

Der erfindungsgemäß erzielte Stabilisierungseffekt unter Verwendung verschiedener Stabilisierungsmittel gemäß der Erfindung geht aus der nachstehenden Tabelle hervor. Sie zeigt den Extinktionsanstieg einer wässrigen Lösung, welche 0,2 mM/l MBTH bzw. MBTH-S, 0,6 mM/l Äthylhydroxytoluidin und 0,1 M/l Kaliumphosphatpuffer pH 7,0 enthält bei 25 °C.

Tabelle 1

| | Zusatz | | Ext.anstieg nach 1 h/25° MBTH | Ext.anstieg nach 1 h/25° MBTH-S |
|---|---|---|---|---|
| 0. | ohne | | 1.060 | 0,126 |
| 1. | Nitrilotrieessigsäure | 1 mM | 0,485 | 0,028 |
| 2. | A | 1 mM | 0,045 | 0,015 |
| 3. | B | 1 mM | 0,045 | 0,015 |
| 4. | C | 1 mM | 0,040 | 0,015 |
| 5. | D | 1 mM | 0,063 | 0,013 |
| 6. | E | 1 mM | 0,052 | 0,013 |
| 7. | Diäthylentriamin | 1 mM | --- | 0,039 |
| 8. | Acetylaceton | 1 mM | 0,581 | 0,030 |
| 9. | Thiosemicarbazid | 1 mM | 0,120 | 0,023 |
| 10. | $K_4[Fe(CN)_6]$ | 1 mM | 0,053 | 0,026 |
| 11. | $NaN_3$ | 1 mM | 0,209 | 0,026 |
| 12. | $K_4[Fe(CN)_6]$ | 0,1 mM | 0,016 | 0,005 |

A = Ethylendinitrilotetraessigsäure (EDTA)
B = Ethylendinitrilotetraessigsäure Dinatriumsalz (EDTA-Na)
C = 1,2 Cyclohexylendinitrilotetraessigsäure
D = Diethylentriaminpentaessigsäure
E = 3,6-Dioxaoctamethylendinitrilotetraessigsäure

Die Werte der obigen Tabelle zeigen die wesentlich überlegene Stabilität des erfindungsgemäßen Reagenz im Vergleich zu einem Reagenz, welches keine der erfindungsgemäß stabilisierenden Substanzen enthält.

Die Bestimmung wurde durchgeführt, indem der jeweiligen Lösung 1 U/ml Peroxidase zugesetzt, die Extinktion gemessen und nach einer Stunde erneut die Extinktion gemessen und die Extinktionsdifferenz bestimmt wird.

Daß das erfindungsgemäße Reagenz auch in Gegenwart einer Oxidase überlegene Stabilität aufweist, zeigt das nachfolgende Beispiel.

Beispiel

Ein erfindungsgemäßes Reagenz, bestehend aus

0,1 Mol Kaliumphosphat, pH 7,0,
0,4 mMol Ethylhydroxitoluidin,
0,2 mMol MBTH-S,
300 U Uricase,
1 000 U Peroxidase,
20 mg Kaliumferrozyanid,
200 mg EDTA-Na$_2$,
100 mg Natriumazid,

wurde in 1 l Wasser aufgelöst und die Extinktion bestimmt. Nach 1,5 stündigem Stehen bei 25 °C wurde die Extinktion erneut bestimmt.

Zum Vergleich wurde ein entsprechendes Reagenz hergestellt, welches jedoch kein Kaliumferrozyanid, EDTA und Natriumazid enthielt.

Das letztere, nicht erfindungsgemäße Reagenz ergab eine Extinktionsänderung von 0,172 E/h. Die entsprechende Änderung beim erfindungsgemäßen Reagenz betrug 0,0072 E/h.

**Ansprüche**

1. Stabilisiertes Reagenz zum Nachweis von $H_2O_2$ oder $H_2O_2$ liefernden Systemen auf Basis von Methylbenzthiazolonhydrazon oder einem Derivat desselben als Farbbildner und eines damit zur oxidativen Kupplung unter Farbentwicklung befähigten aromatischen Amins, enthaltend Puffersubstanz und Peroxidase, gekennzeichnet durch einen Gehalt an wenigstens einer Substanz aus der Gruppe Alkaliferrozyanid, Chelatbildner und Alkaliazid als Stabilisator sowie gegebenenfalls einer Oxidase.

2. Reagenz nach Anspruch 1, dadurch gekennzeichnet, daß es Methylbenzthiazolonhydrazonsulfonat enthält.

3. Reagenz nach Anspruch 1 oder 2, dadurch gekennzeichnet, daß es als aromatisches Amin ein m-Toluidin enthält.

4. Reagenz nach einem der Ansprüche 1 bis 3, dadurch gekennzeichnet, daß es das Alkaliferrozyanid als Kaliumsalz enthält.

5. Reagenz nach einem der vorhergehenden Ansprüche, dadurch gekennzeichnet, daß es als Chelatbildner ein Aminpolyazetat enthält.

6. Reagenz nach Anspruch 5, dadurch gekennzeichnet, daß es pro Liter gebrauchsfertige Lösung 1 bis 500 mg Kaliumferrozyanid, 1 bis 1 000 mg Ethylendiamintetraazetat und 5 bis 500 mg Natriumazid enthält.

7. Reagenz nach Anspruch 6, dadurch gekennzeichnet, daß es pro Liter gebrauchsfertige Lösung 10 bis 50 mg Kaliumferrozyanid, 100 bis 1 000 mg Ethylendiamintetraazetat und 50 bis 500 mg Natriumazid enthält.

8. Reagenz nach einem der Ansprüche 1 bis 7, dadurch gekennzeichnet, daß es auf einem saugfähigen Trägermaterial imprägniert vorliegt.

9. Verfahren zum Nachweis von $H_2O_2$ oder $H_2O_2$ liefernden Systemen, dadurch gekennzeichnet, daß man der zu untersuchenden Lösung ein Reagenz nach einem der Ansprüche 1 bis 8 zusetzt und die gebildete Färbung mißt.

**Claims**

1. Stabilised reagent for the detection of $H_2O_2$ or of $H_2O_2$-forming systems based upon methylbenzothiazolone hydrazone or a derivative thereof as colour former and an aromatic amine capable of undergoing oxidative coupling therewith with colour development, containing buffer substance and peroxidase, characterised by a content of at least one substance from the group alkali metal ferrocyanide, chelate former and alkali metal azide as stabiliser, as well as optionally an oxidase.

2. Reagent according to claim 1, characterised in that it contains methylbenzothiazolone hydrazone sulphonate.

3. Reagent according to claim 1 or 2, characterised in that as aromatic amine it contains an m-toluidine.

4. Reagent according to one of claims 1 to 3, characterised in that it contains the alkali metal ferrocyanide as potassium salt.

5. Reagent according to one of the preceding claims, characterised in that it contains an aminopolyacetate as chelate former.

6. Reagent according to claim 5, characterised in that, per litre of ready-to-use solution, it contains 10 to 50 mg potassium ferrocyanide, 10 to 1 000 mg ethylenediamine-tetraacetate and 50 to 500 mg sodium azide.

7. Reagent according to claim 6, characterised in that, per litre of ready-to-use solution, it contains 10 to 50 mg potassium ferrocyanide, 100 to 1 000 mg ethylenediamine-tetraacetate and 50 to 500 mg sodium azide.

8. Reagent according to one of claims 1 to 7, characterised in that it is present impregnated on an absorbent carrier material.

9. Process for the detection of $H_2O_2$ or of $H_2O_2$-forming systems, characterised in that to the solution to be investigated one adds a reagent according to one of claims 1 to 8 and measures the colour formed.


**Revendications**

1. Réactif stabilisé pour la détection de $H_2O_2$ ou de systèmes fournissant $H_2O_2$ à base de méthylbenzthiazolonehydrazone ou d'un dérivé de celle-ci comme chromogène et d'une amine aromatique susceptible de couplage oxydant avec celle-ci avec développement de couleur, contenant de la substance tampon et de la peroxydase, caractérisé par une teneur en au moins une substance du groupe ferrocyanure alcalin, chélatant et azide alcalin comme stabilisant, ainsi que le cas échéant en une oxydase.

2. Réactif suivant la revendication 1, caractérisé en ce qu'il contient du sulfonate de méthylbenzthiazolonehydrazone.

3. Réactif suivant la revendication 1 ou 2, caractérisé en ce qu'il contient, comme amine aromatique, une m-toluidine.

4. Réactif suivant l'une des revendications 1 à 3, caractérisé en ce qu'il contient le ferrocyanure alcalin sous forme de sel de potassium.

5. Réactif suivant l'une des revendications précédentes, caractérisé en ce qu'il contient, comme chélatant, un polyacétate d'amine.

6. Réactif suivant la revendication 5, caractérisé en ce qu'il contient, par litre de solution prête à l'emploi, 1 à 500 mg de ferrocyanure de potassium, 1 à 1 000 mg d'éthylènediaminetétracétate et 5 à 500 mg d'azide de sodium.

7. Réactif suivant la revendication 6, caractérisé en ce qu'il contient, par litre de solution prête à l'emploi, 10 à 50 mg de ferrocyanure de potassium, 100 à 1 000 mg d'éthylènediaminetétracétate et 50 à 500 mg d'azide de sodium.

8. Réactif suivant l'une des revendications 1 à 7, caractérisé en ce qu'il est présent imprégné sur une matière de support absorbante.

9. Procédé de détection d'$H_2O_2$ ou de systèmes fournissant $H_2O_2$, caractérisé en ce qu'on ajoute à la solution à étudier un réactif suivant l'une des revendications 1 à 8, et en ce qu'on mesure la coloration formée.